(19) Europäisches Patentamt
European Patent Office
Office européen des brevets

(11) **EP 2 136 737 B1**

(12) **EUROPEAN PATENT SPECIFICATION**

(45) Date of publication and mention
of the grant of the patent:
**06.03.2013 Bulletin 2013/10**

(51) Int Cl.:
***A61F 2/14*** (2006.01)

(21) Application number: **08742784.5**

(86) International application number:
**PCT/US2008/004711**

(22) Date of filing: **11.04.2008**

(87) International publication number:
**WO 2008/127653 (23.10.2008 Gazette 2008/43)**

(54) **ARTIFICIAL CORNEA**

KÜNSTLICHE HORNHAUT

CORNÉE ARTIFICIELLE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **13.04.2007 US 735179**

(43) Date of publication of application:
**30.12.2009 Bulletin 2009/53**

(73) Proprietor: **Gore Enterprise Holdings, Inc.
Newark, DE 19714-9206 (US)**

(72) Inventors:
• **AKPEK, Esen, K.
Baltimore, MD 21209 (US)**
• **BALAJI, Gopalan, V.
Kennett Square, PA 19348 (US)**
• **FISCHER, Paul, J.
Greenville, DE 19807 (US)**
• **SCHMIEDEL, Thomas, B.
Bear, DE 19701 (US)**
• **SINGH, Anuraag
Newark, DE 19711 (US)**

(74) Representative: **Shanks, Andrew
Marks & Clerk LLP
Aurora
120 Bothwell Street
Glasgow
G2 7JS (GB)**

(56) References cited:
**EP-A- 1 498 087          WO-A-97/27824
WO-A-2006/009490      FR-A- 2 649 605
US-A1- 2002 007 217**

**Description**

**FIELD OF THE INVENTION**

The present invention relates to artificial corneas for restoring vision to patients with diseased or otherwise damaged or non-functioning corneas.

**BACKGROUND OF THE INVENTION**

Keratoprosthesis, or artificial cornea, development has been ongoing for almost 200 years. The primary challenges facing keratoprostheses as a means to address reversible blindness has been the lack of biointegration or the extrusion of the device from the eye. Device rejection is often observed to initiate as tissue melting around the prosthesis followed by extrusion. Other complications that have hampered keratoprosthesis efficacy include infection, retroprosthetic membrane formation, inflammation, glaucoma, lack of mechanical durability and optical fouling.

Numerous device constructions utilizing a variety of material sets have been investigated. Most recently, two main types of devices have been the subject of research interest. The first type is typically referred to as a "collar-button" type keratoprosthesis and the second type utilizes a "core and skirt" morphology.

The Dohlman-Doane keratoprosthesis, available from the Massachusetts Eye and Ear Institute, is an example of the collar-button type device. It consists of a PMMA optic screwed into a plate and locking ring. The device integrates with the host eye via a donor cornea sandwiched between the optic and the plate.

Prior art constructions have typically involved keratoprostheses of the core and skirt type construction. The core and skirt type devices generally have a non-porous optical core for visual restoration and a microporous skirt for bio-integration within the eye. Examples of polymers described as useful for the optic core include PMMA, silicones, hydrogels and polyurethanes. Carbon fibers, PTFE, expanded PTFE, polyethylene terephthalate, polyesters, polyurethanes, polypropylene and hydrogels are some of the polymers that have been employed in the porous skirt. The AlphaCor KPro, currently available from Addition Technology, Inc., is an example of a keratoprosthesis of the core and skirt construction.

Many core and skirt constructions have been contemplated in the prior art. For example, U.S. Patent No. 5,282,851, to LaBarre, describes an intraocular prosthesis for implanting in an annular void extending radially into the tissues of the eye. The prosthesis is described as a circular, transparent, elastomeric optical element having a peripheral edge and a non-rigid or flexible porous skirt which encircles the element and extends radially from the periphery of the element, extending radially into the annular void of the eye when positioned in a patient. The prosthesis may also include spoke-like porous extensions extending from the skirt.

U. S. Patent No. 5,843,185, issued to Leon Rolden et al., describes a keratoprosthesis containing an optical support segment including a disc like configuration and is constructed of hydroxyapatite to promote cell growth, increased vascularization and/or bone formation therein so as to substantially increase the optical support segment's long term assimilation with the patient's eyeball, combat infection and heal quickly. Disposed in the optical aperture of the optical support segment is an optical cylinder which is preferably removable and provides for the transmittal of light into the eyeball.

U. S. Patent No. 5,489,301, issued to Barber, describes a corneal prosthesis with an optical core member having a frustoconical shape and preferably a substantially cylindrical anterior section and a substantially conical posterior section. The optical core member is made of an alloplastic material, preferably intraocular grade PMMA, and has an anterior flange portion extending peripherally about the anterior optical end portion of the core member. A skirt member, described as a hydrophilic porous semi-flexible material, extends peripherally about and is attached, preferably mechanically, to the optical core member at a peripheral groove between the anterior section and the posterior section and is capable of being implanted in a lamellar pocket in the eye.

U. S. Patent No. 6,391,055, issued to Ikada et al., describes an artificial cornea comprising an optical element made of an optically transparent material, having a front surface and a posterior surface, and a skirt provided so as to support at least a part of the optical element, wherein the skirt has a flange on its side facing the interior of the eye, and the flange radially protrudes outward from the skirt.

U. S. Patent No. 5,300,116, issued to Chirila et al., describes a composite device for implanting in the cornea of the human eye consisting of a transparent central portion intimately attached to an opaque spongy rim. Both portions are made of hydrogel materials produced in different conditions of polymerization during a two-stage process performed in a specific molding unit.

U. S. Patent No. 6,976,997, issued to Noolandi et al., describes two-phase and three-phase artificial corneas having a flexible, optically clear central core and a hydrophilic, porous skirt, both of which are biocompatible and allow for tissue integration. The three-phase artificial cornea further has an interface region between the core and the skirt.

International Patent Application Publication No. 2004-0024448, published on February 5, 2004, describes medical devices provided with at least a partial surface coating or component of a thermoplastic copolymer of tetrafluoroethylene and perfluoroalkylvinylether that is free of cross-linking monomers and curing agents. The fluoropolymer component is preferably an amorphous thermoplastic, is highly inert and biocompatible, and has elastomeric characteristics that provide desirable mechanical properties such as good flexibility and durability. In the case of artificial corneas or keratoprostheses, devices are described having porous peripheral skirts attached to a fluoropolymer cornea substitute component. The skirts have a porosity that can be tailored to promote rapid ingrowth and attachment into surrounding native tissue, and the cornea substitute layer can be shaped to conform to surrounding native tissue and have a thickness, flexibility and creep resistance suitable for long term ocular implantation.

International Patent Application Publication No. 2005-0129735, published on Jun. 16, 2005, describes implantable medical devices including a porous membrane that is treated with a hydrophilic substance. Advantages of the hydrophilic substance include the ability to achieve, for example, rapid optimum visualization using technology for viewing inside of a mammalian body. In particular for keratoprosthetic medical devices, the hydrophilic substance was described as enhancing corneal epithelial attachment to the keratoprosthesis.

International Patent Application Publication No WO 97/27824 discloses a corneal prosthesis device positionable in the cornea. The device includes a substantially cylindrical optical portion (10) made of a first transparent material and having a front optical dioptric surface (14) and a rear end (16), a front ring-shaped skirt (18) projecting from the side wall of the optical portion and made of biocompatible and biocolonisable polymer that is different from the first material, and a rear ring-shaped skirt (20) projecting from the side wall of the optical portion and made of said first material.

Despite the wide range of devices taught and contemplated by the prior art, significant issues still exist with keratoprosthesis design to achieve optimum device anchoring and, ultimately, long-term patency.

## SUMMARY OF THE INVENTION

According to an aspect of the present invention, there is provided an artificial cornea according to the appended claims. The artificial cornea may be designed to restore vision in patients with diseased or otherwise damaged or non-functioning corneas. As used herein, the term "artificial cornea" is intended to include all forms of artificial cornea, including synthetic corneas, keratoprostheses and the like. Solely for convenience, these terms may be used interchangeably throughout the present specification. The present device construction, in one embodiment, comprises a biocompatible, non-porous fluoropolymer optic component bonded to porous layers of biocompatible polymeric material in unique constructions heretofore not contemplated in the prior art.

Specifically, the artificial cornea of the present invention comprises, in one embodiment, a polymeric corneal substitute material comprising a disk with an anterior surface and a posterior surface, said disk having a first protrusion on said anterior surface forming or having thereon an optical surface. As used herein, the term "disk" is intended to refer to a substantially circular or elliptical shape which may be flat or have some curvature (e.g., whether concave, tapered, etc.). The outer perimeter, or edge, of the disk may be regular or irregular (e.g., scalloped, spoked, star-shaped, etc.). As used herein, the term "protrusion" is intended to refer to a region which protrudes, projects or otherwise extends above or beyond the normal contour or surface of the disk, the normal disk contour or surface being defined by substantially parallel or partially tapered lines which can be drawn on a disk cross-section extending from one side to the opposite side of the disk along each of the anterior and posterior surfaces and following the normal or surface contour of the disk, whether straight, curved, etc. Optionally, a second protrusion may be oriented on the posterior surface. The artificial cornea further includes a mechanical anchoring means for anchoring the artificial cornea in the patient, and a unique sealing region around the perimeter of the optical surface. In one embodiment of the present invention, the artificial cornea of the present invention comprises a polymeric corneal substitute material comprising a disk with an anterior surface and a posterior surface, said disk having a first protrusion on said anterior surface forming or having thereon an optical surface and a second protrusion on said posterior surface, a first annular layer having an inner opening oriented on the anterior surface of said disk so that the inner opening of the first annular layer is oriented around said first protrusion, and a second annular layer having an inner opening, said second annular layer oriented on the posterior surface of said disk so that the inner opening of the second annular layer is oriented around said second protrusion, and a sealing region around the perimeter of the optical surface.

As used herein, the term "optical surface" is intended to refer to a surface which significantly contributes to the formation of an image in the scope of visual acuity by being the primary refractive surface in the optical path. The optical surface is the interface between the artificial cornea and the external environment and is located on at least a portion of the first protrusion. The optical surface substantially participates in the formation of a clear, distortion-free image. The optical surface also is capable of high light transmission and is ideally largely free of surface defects like scratches or other imperfections. The optical surface may be formed as part of the polymeric corneal substitute material, whether or not subjected to any desired surface treatment, or may be a separate material oriented or coated thereon.

The posterior and anterior porous annular rings are capable of being sutured during surgery to mechanically anchor, or hold, the device in place. The sutures provide mechanical anchoring of the device after the implantation surgery. Subsequent attachment of cells to the porous annular rings provides mechanical anchoring of the device.

The first annular layer comprises a first porous polymeric material and is located adjacent the anterior surface of said disk so that the inner opening of the annular layer is oriented around said first protrusion. The second annular layer comprises a second porous polymeric material and is oriented adjacent the posterior surface of said disk so that the inner opening of the annular layer is oriented around said second protrusion. These annular layers permit suture retention and allow for mechanical attachment of cells, and can enhance the mechanical anchoring of the device in the patient.

A novel feature of the present invention is the unique sealing region provided at least around the perimeter of the optical surface. The sealing region is made of a material which is capable of allowing mechanical attachment of cells and sealing against ingress of bacteria (thus, reducing chance of infection). Thus, the present invention satisfies a long-standing need by providing a unique combination of a synthetic, optically clear, easily implantable, durable artificial cornea which allows attachment of cells and which provides a unique sealing region to create a desirable surface seal of the device upon implantation.

Depending on the specific performance requirements of the device, the sealing region can be either porous or non-porous. For example, in embodiments where the sealing region is porous, the porosity may be either uniformly or non-uniformly distributed in the sealing region, and the sizes of the pores may be uniform or non-uniform, again depending on the specific performance requirements of the device. In embodiments wherein the sealing region is non-porous, the sealing region may comprise a porous structure where the porosity is filled with a second material, such as a biodegradable material, one or more drugs, cell growth enhancers, or the like. Alternatively, the sealing region may be non-porous with some suitable surface modification, such as a surface roughness or other feature which allows mechanical attachment of cells thereto.

In one embodiment of the present invention, this sealing region is uniquely oriented at the perimeter, or edge, of the optical surface to facilitate biointegration of the artificial cornea and sealing against infection. In an alternate embodiment of the invention, the sealing region can be provided by a material which covers the entire optical surface, whereby the sealing material allows cell attachment and seals against ingress of bacteria.

As noted above, the unique sealing region of the present invention is oriented at least at the perimeter of the optical surface and is capable of sealing against ingress of bacteria (thus, reducing chance of infection) and allowing cell attachment (i.e., facilitates biointegration). The sealing region can be porous or nonporous, depending on the specific performance requirements of the artificial cornea. The sealing region, upon implantation and biointegration of the device, facilitates separation of the intra-ocular sterile space from the environment and attendant contamination. It also affords a zone amenable to accommodate small changes in the final diameter of the trephined cornea (i.e., the corneal material which remains after removal of the central corneal circle during surgery) and/or variations in positioning of the device during surgery, while still providing a sealing functionality. Additionally, the location of the sealing region adjacent to the optical surface minimizes the possibility of trough formation (e.g., at the edge of the optical surface) which could trap fluids that can cause infection while lingering. Moreover, the sealing region can be tailored to provide special functionality to enhance attachment of, for example, epithelial cells. In an alternative embodiment, an extended sealing region over the entire optical surface can aid in the complete integration of the device into the body, while still allowing for superior optical transmission via attachment of cells to form a surface similar to the natural corneal surface. Once a cell layer has penetrated the sealing region in depth, the extended sealing region will have a refractive index similar to that of the natural cornea, and thus the sealing region will become the first refractive surface, as is the case with a natural cornea present in a healthy eye. The optical quality of this sealing region penetrated with cells will depend on a close match of refractive index to that of the cornea.

The polymeric corneal substitute material incorporated in the disk can comprise fluoropolymers selected from a copolymer of tetrafluoroethylene (TFE) and perfluoroalkylvinylether, a copolymer of tetrafluoroethylene and hexafluoropropylene (FEP), perfluoropolymers preferably containing TFE as a comonomer, PFA, perfluoropolyethers, or alternatively, can comprise silicone, poly(methyl methacrylate) (PMMA), hydrogel, polyurethane, or any appropriate suitable combinations thereof. The polymeric corneal substitute layer preferably has a refractive index in the range of 1.2 to 1.6, more preferably 1.3 to 1.4. It is further preferred that the polymeric corneal substitute layer have a light transmission in the visible light transmission range (wavelength of from 400-700 nm) of greater than 50%, more preferably greater than 80%. The polymeric corneal substitute material may be porous or non-porous depending on the desired end application of the device. Additives such as cross-linking agents, biologically active substances (e.g., growth factors, cytokines, heparin, antibiotics or other drugs), hormones, ultraviolet absorbers, pigments, other therapeutic agents, etc., may be incorporated depending on the desired performance of the device. One example of a suitable fluoropolymer for the device is described in International Patent Application Publication No. 2004-0024448, discussed earlier herein. As described in that publication, the fluoropolymer may optionally contain various additives. The thermoplastic fluoropolymer is a copolymer of tetrafluoroethylene (TFE) and perfluoroalkylvinylether (PAVE) that is free of cross-linking monomers and curing agents. The perfluoroakylvinylether may be perfluoromethylvinylether (PMVE), perfluoroethylvinylether (PEVE) or perfluoropropylvinylether (PPVE). The desirable mechanical characteristics, particularly tensile strength and toughness, are surprising given the absence of cross-linking monomers, curing agents, and process aids and fillers that would otherwise render such materials inadequately biocompatible. The copolymer of TFE and PMVE is generally preferred, and may be made by emulsion polymerization techniques. The PMVE content ranges from 40 to 80% by weight, while the complemental TFE content ranges from 60 to 20% by weight.

The first and second annular layers can be made from any materials which permit suture retention and allow for mechanical attachment of cells to provide mechanical anchoring of the device in the patient. One example of a suitable material for the annular layers of the invention is expanded polytetrafluoroethylene (ePTFE). This material has a porosity that can be tailored by a variety of means to promote mechanical attachment of cells, and thus attachment to surrounding native tissue. Other suitable materials, whether porous or non-porous, are also contemplated to be within the scope of the invention, provided they permit suture retention and allow for mechanical attachment of cells. For example, in embodiments where the annular layers are porous, the porosity may be either uniformly or non-uniformly distributed, and the sizes of the pores may be uniform or non-uniform, again depending on the specific performance requirements of the device. In embodiments wherein the annular layers are non-porous, the layers may comprise a porous structure where the porosity is filled with a second material, such as a biodegradable material, one or more drugs, cell growth enhancers, or the like. Alternatively, the annular layers may be non-porous with some suitable surface modification, such as surface features which provide roughness and which allow mechanical attachment of cells thereto. Additionally, the geometry of the device may be tailored to achieve desired effects - e.g., openings or through-paths, porosity, patterns, etc., may be incorporated in (1) the polymeric corneal material only; or (2) the polymeric corneal material and one or both of the annular layers to achieve a variety of effects. For example, such openings may, among other effects, enhance nourishment or hydration of the lamellae after implantation.

Materials suitable for the sealing region of the present invention include those with a structure which is capable of accommodating mechanical attachment of cells. Alternatively, in an embodiment where the sealing region extends across the optical surface, it is desirable that the sealing region be made of a material which, when cells attach thereto, reaches a desirable level of transparency and is substantially free of optical distortion. One example of a material suitable for the sealing region is ePTFE. As noted earlier with respect to ePTFE, this material has a porosity that can be tailored by a variety of means to allow cell attachment. Other suitable materials, whether porous or non-porous, are also contemplated to be within the scope of the invention, provided they allow cell attachment and/or anchoring. Additionally, depending on the particular desired characteristics of the device, the artificial cornea may optionally be treated to render it hydrophilic.

Accordingly, the present invention satisfies a long-standing need by providing a unique combination of a synthetic, optically clear, easily implantable, durable artificial cornea which is readily biointegratable and which provides a unique sealing region to create a desirable surface seal of the device upon implantation.

It will be apparent from the following detailed description and the examples contained herein that this invention lends itself to a wide variety of material combinations, device geometries and orientations which are within the contemplated scope of the invention.

## DESCRIPTION OF THE DRAWINGS

The operation of the present invention should become apparent from the following description when considered in conjunction with the accompanying drawings, in which:

Figures 1 a and 1 b are schematic cross-sectional views of embodiments of a device of the present invention;
Figure 2 is a schematic cross-sectional view of another embodiment of a device of the present invention incorporating a reinforcemement within the polymeric corneal substitute material;
Figure 3 is a schematic cross-sectional view of a further embodiment of a device of the present invention;
Figure 4 is a schematic cross-sectional view of another embodiment of a device of the present invention;
Figure 5 is a schematic cross-sectional view of another embodiment of a device of the present invention;
Figure 6 is a schematic cross-sectional view of another embodiment of a device of the present invention having a sealing material which extends across the optical surface;
Figure 7 is a schematic cross-sectional view of a further embodiment of a device of the present invention; and
Figures 8a and 8b are top perspective views of devices of the present invention showing an embodiment of the present invention with alternative orientations of openings, or through-paths, therein.

## DETAILED DESCRIPTION OF THE INVENTION

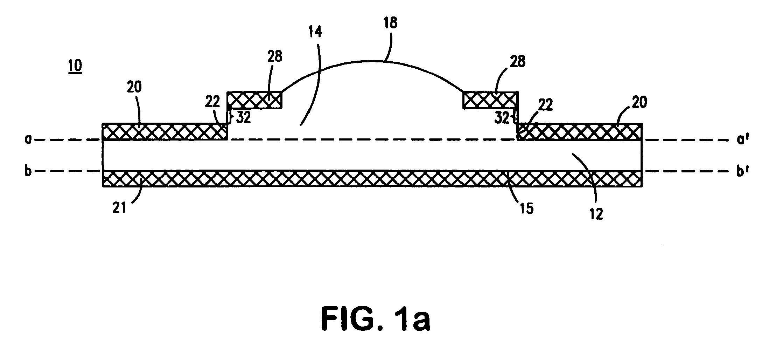

Referring to Figure 1a, there is shown a schematic representation of a first embodiment of an artificial cornea of the present invention. Specifically, the artificial cornea 10 comprises a polymeric corneal substitute material in the form of a disk 12 having an anterior protrusion 14 (extending above the dashed line a-a'), a posterior surface 15 (extending along the dashed line b-b') and an optical surface 18 on at least a portion of the anterior protrusion 14. An annular attachment layer of polymeric material 20 is oriented adjacent the disk 12 so that the inner opening 22 of the annular layer 20 is oriented around the anterior protrusion 14. An additional attachment layer of polymeric material 21 is oriented adjacent the posterior surface 15 of the disk 12. Depending on the desired function, the annular attachment layer 20 and the additional attachment layer 21 may be the same or different in composition and/or structure. As used herein, the term "annular" is intended to include any circular, elliptical, scalloped, star-shaped, spoke-like, or other suitable geometry for the outer perimeter of the component.

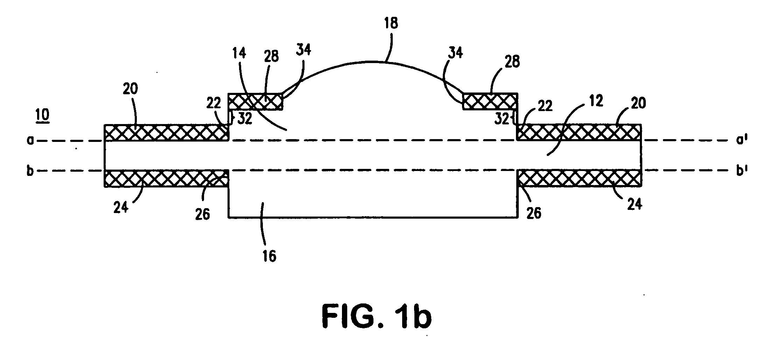

Referring to Figure 1b, there is shown a schematic representation of an alternative embodiment of an artificial cornea of the present invention. Specifically, the artificial cornea 10 comprises a polymeric corneal substitute material in the form of a disk 12 having an anterior protrusion 14 (extending above the dashed line a-a'), a posterior protrusion 16 (extending below the dashed line b-b') and an optical surface 18 on at least a portion of the anterior protrusion 14. A first annular attachment layer of polymeric material 20 is oriented adjacent the disk 12 so that the inner opening 22 of the annular layer 20 is oriented around the anterior protrusion 14. A second annular attachment layer of polymeric material 24 is oriented adjacent the disk 12 so that the inner opening 26 of the annular layer 24 is oriented around the posterior protrusion 16. Depending on the desired function, the first and second annular attachment layers may be the same or different in composition and/or structure. As used herein, the term "annular" is intended to include any circular, elliptical, scalloped, star-shaped, spoke-like, or other suitable geometry for the outer perimeter of the component.

In the embodiments depicted in Figures 1a and 1b, a sealing region 28 comprising a polymeric material is oriented on the anterior protrusion 14 so that the inner opening 34 of the sealing region 28 is around the optical surface 18. In these embodiments, an offset 32 is present in the anterior protrusion 14 between the first annular attachment layer 20 and the sealing region 28.

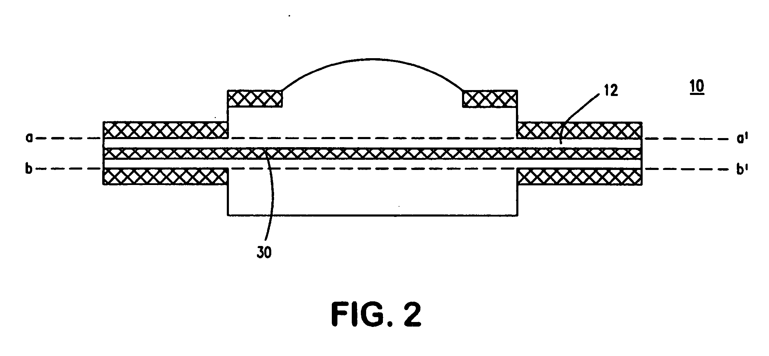

In an alternative embodiment of the present invention, referring to Figure 2, there is shown another embodiment of the present invention, wherein a reinforcing member 30 is included within the disk 12. The inclusion of such a reinforcing member may be desirable to provide mechanical support to the artificial cornea. The reinforcing member may be oriented across the entire disk, as shown, or alternatively may be oriented only in selected regions within the disk. The reinforcing member may comprise any suitable material which provides some mechanical support to the artificial cornea. In one embodiment, the reinforcing member may have the same composition as one or more of the annular attachment layers.

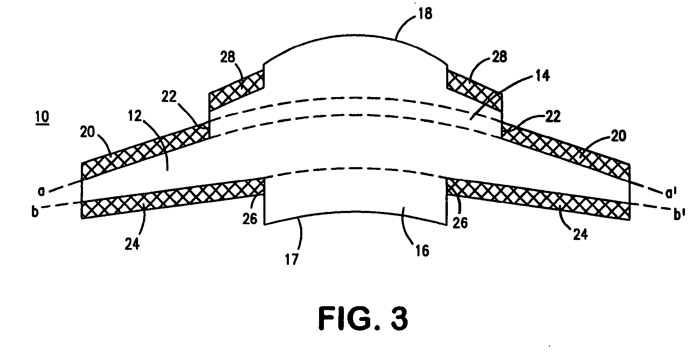

Depending on the desired final geometry of the artificial cornea of the present invention, it will be apparent to one of skill in the art that any suitable geometries, perimeter shapes, flat planes, curvatures, tapers, patterns, etc., may be employed in the unique construction of the present invention. Referring to Figure 3, for example, this Figure shows an embodiment of an artificial cornea of the invention wherein the disk 12 narrows in a tapered geometry toward its outer perimeter. Additionally, the posterior protrusion 16 has a concave geometry to its surface 17, which may be desirable in certain instances to assist in implanting the device (e.g, foldability, etc.), focusing of an image, etc.

The geometry of the anterior protrusion of the device may also be tailored to achieve a variety of suitable configurations of the device of the present invention. For example, in Figures 1-3, described above, the anterior protrusion is shown as having a stepped configuration, wherein an offset (shown as 32 in Figures 1a and 1b) exists between the the first annular attachment layer 20 and the sealing region 28, and the sealing region 28 is positioned on the anterior protrusion around the perimeter of the optical surface 18.

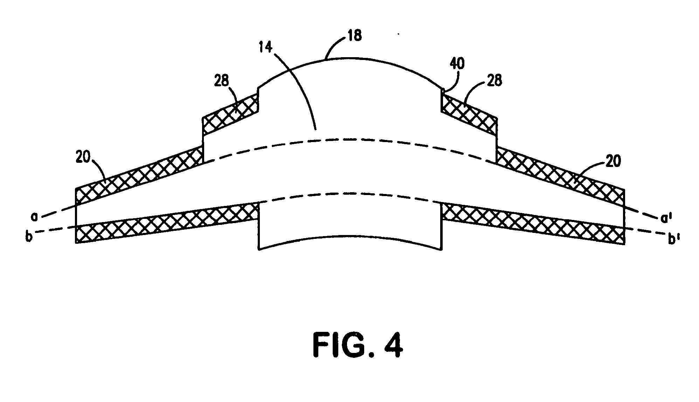

Figure 4 depicts and alternative embodiment of the artificial cornea device of the present invention, wherein substantially no offset exists in the anterior protrusion 14 between the first annular attachment layer and the sealing region. Depending on the specific requirements of the particular design, the first annular attachment layer and the sealing region may or may not touch one another. Figure 4 does, alternatively, show an optional offset 40 between the inner opening 34 of the sealing region 28 and the optical surface 18. Again, depending on the specific requirements of a particular end application, the geometry of the device may be tailored in a variety of ways which are contemplated to be within the scope of the present invention.

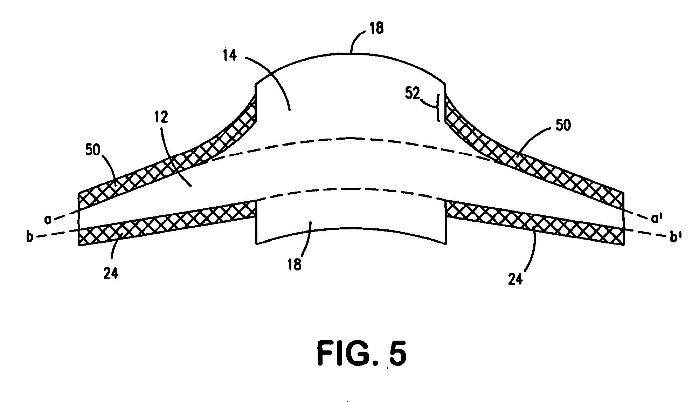

In a further embodiment of the present invention, the first annular attachment layer and the sealing region may comprise a single continuous attachment and sealing material 50, as depicted in Figure 5, wherein the material is positioned on the disk and the anterior protrusion so that it extends toward and in close proximity to the perimeter of the optical surface. The inner opening 52 of the continuous attachment and sealing material 50 may optionally have a tapered configuration, as shown.

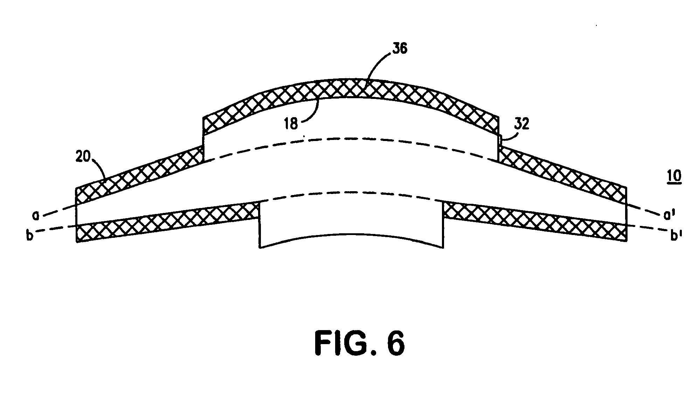

In a further alternative embodiment of the present invention, as described earlier herein, the artificial cornea may comprise a sealing region which completely covers the optical surface of the artificial cornea and allows complete integration of the device into the body. Referring to Figure 6, there is shown such an embodiment of the present invention, wherein a sealing region 36 comprising a porous polymeric material extends across the entire optical surface 18 of the artificial cornea 10. An offset 32 is shown in the anterior protrusion 14 between the sealing region 36 and the first annular attachment layer 20.

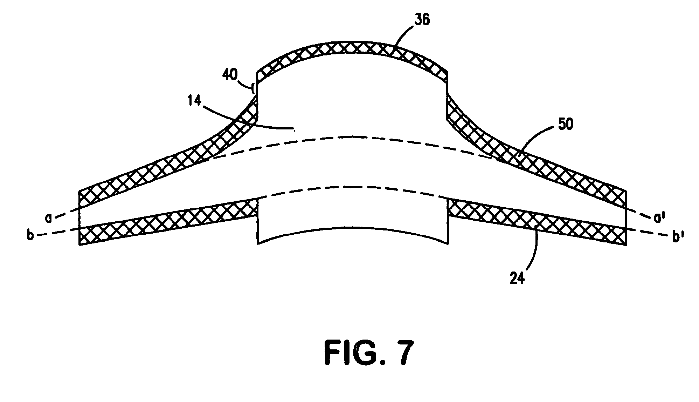

Figure 7 shows a further alternative embodiment of the device of the present invention, wherein the sealing region 36 extends across the optical surface 18 and the continuous attachment and sealing layer 50 is oriented on the disk 12 and extends along at least a portion the anterior protrusion 14. In this embodiment, the sealing region extends substantially to the optical surface, and the gradual taper shown can be beneficial upon implantation by reducing the potential for gap or trough creation (thus, limiting sealing) relative to a device configuration with a stepped geometry. An optional offset, shown as 40, may exist between the continuous attachment and sealing layer 50, but such an offset is merely optional depending on the desired final configuration of the device.

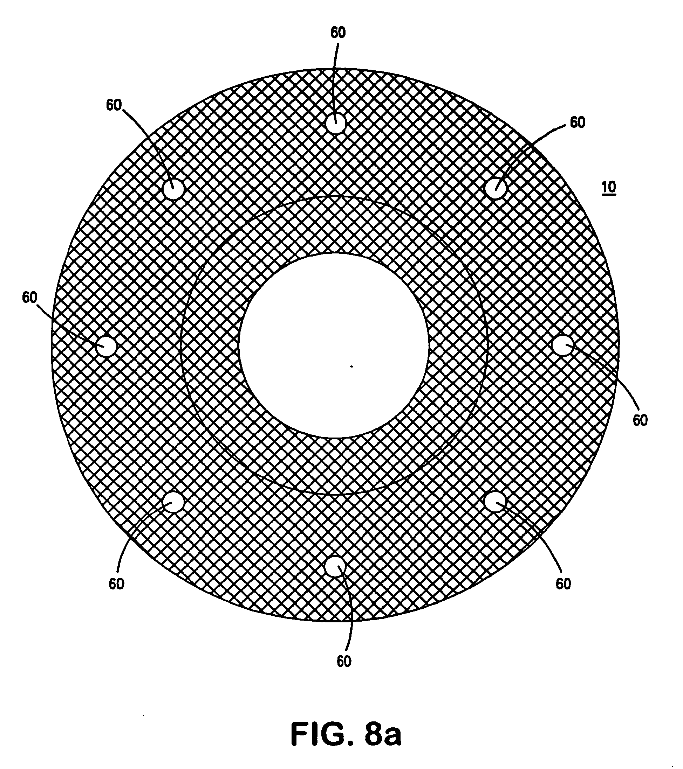

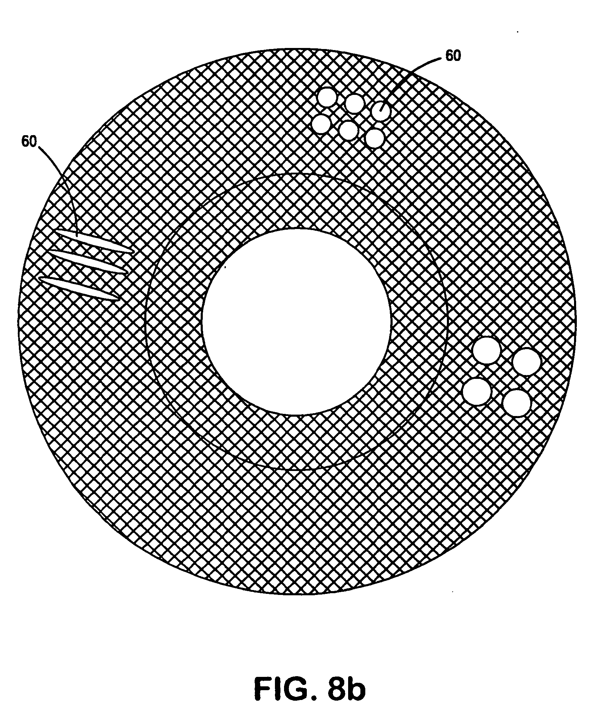

As described earlier herein, the artificial corneas of the present invention may be tailored to include openings or through-paths, porosity, patterns, etc., in (1) the polymeric corneal material only; or (2) the polymeric corneal material and one or both of the annular layers to achieve a variety of effects. Figure 8a and 8b are top perspective views of alternative embodiments of devices of the present invention showing alternative orientations and geometries of openings, or through-paths, therein. For example, Figure 8a shows a device of the invention 10, wherein openings 60 are substantially uniformly distributed around the device. Alternatively, Figure 8b shows alternative orientations and geometries of the openings 60. Again, the specific configuration and orientation of openings in the devices of the present invention will depend on the desired effects in the device.

## TEST METHODS

### Particle Size Determination

A COULTER N4MD particle size analyzer was used. The mean diameter was measured using a light scattering method with helium laser at scattering angles of 90 degrees. Each aqueous dispersion sample was diluted about 10,000 times with deionized water before measurement.

### Mean Tensile Strength and 100% Secant Modulus (for Polymer)

Tensile tests were performed under ambient conditions (23 +/- 1 °C) using a tensile testing machine (Instron Model 5564, Norwood, MA) equipped with an Instron 2603-080 extensometer for strain measurement. Testing was performed on laser-cut dogbone-shaped test samples with a gauge length of 40mm. Test samples were measured for thickness using a Starrett No. 1015MB-881 0.01 mm snap gauge and for width using a Peak 10x scaled lupe; test samples were then conditioned for a minimum of one hour at ambient conditions (23 +/- 1°C) prior to testing. Testing was performed at a cross-head speed of 250 mm/min. Tensile strength and secant modulus values at 100% elongation were obtained using the Merlin Testing Software V4.42 package that was supplied with the testing machine. Values represent the average of eight measurements.

### Density

Samples die cut to form rectangular sections 2.54 cm by 15.24 cm were measured to determine their mass (using a Mettler-Toledo analytical balance Model AG204) and their thickness (using a Kafer FZ1000/30 snap gauge). Using these data, density was calculated with the following formula:

$$\rho = \frac{m}{w * l * t}$$

in which: p = density (g/cc); m = mass (g); w = width (cm); I = length (cm); and t = thickness (cm). The average of the three measurements was used.

### Tensile Break Load Measurements and Matrix Tensile Strength (MTS) Calculations (for Membranes)

Tensile break load was measured using an INSTRON 5567 tensile test machine equipped with flat-faced grips (one side rubber and the other side serrated) and a 0.5 kN load cell. The gauge length was 5.08 cm and the cross-head speed was 50.8 cm/min. The sample dimensions were 2.54 cm by 15.24 cm. For longitudinal MTS measurements, the larger dimension of the sample was oriented in the machine, or "down web," direction. For the transverse MTS measurements, the larger dimension of the sample was oriented perpendicular to the machine direction, also known as the cross web direction. The thickness of the samples was taken using the Kafer FZ1000/30 thickness snap gauge. Subsequently, each sample was weighed using a Mettler Toledo Scale Model AG204. The samples were then tested individually on the tensile tester. Three different sections of each sample were measured. The average of the three maximum load (i.e., the peak force) measurements was used. The longitudinal and transverse MTS were calculated using the following equation:

$$MTS = (maximum\ load\ /cross\text{-}section\ area)*(bulk\ density\ of\ PTFE)/$$

$$density\ of\ the\ porous\ membrane),$$

wherein the bulk density of PTFE is taken to be 2.2 g/cc.

Thickness

Membrane thickness was measured by placing the membrane between the two plates of a Kafer FZ1000/30 thickness snap gauge (Käfer Messuhrenfabrik GmbH, Villingen-Schwenningen, Germany). The average of three measurements was used.

Mass Per Unit Area

Samples die cut to form rectangular sections 2.54 cm by 15.24 cm were measured to determine their mass (using a Mettler-Toledo analytical balance Model AG204). The mass per unit area was determined by dividing the mass of the sample by the surface area (38.71 cm$^2$).

Ball Burst Strength Measurements

The test method and related sample mounting apparatus were developed by W.L. Gore & Associates, Inc. for use with a Chatillon Test Stand. The test measures the burst strength of materials such as fabrics (woven, knit, nonwoven, etc.), porous or nonporous plastic films, membranes, sheets, etc., laminates thereof, and other materials in planar form.
A specimen was mounted taut, but unstretched, between two annular clamping plates with an opening of 7.62 cm diameter. A metal rod having a polished steel 2.54 cm diameter ball-shaped tip applied a load against the center of the specimen in the Z-direction (normal to the X-Y planar directions). The rod was connected at its other end to an appropriate Chatillon force gauge mounted in a Chatillon Materials Test Stand, Model No.TCD-200. The load was applied at the rate of 25.4 cm/minute until failure of the specimen occurred. The failure (tearing, burst, etc.) may occur anywhere within the clamped area. Results were reported as the average of three measurements of the maximum applied force before failure.
Testing was done at ambient interior temperature and humidity conditions, generally at a temperature of 21 to 24°C and relative humidity of 35 to 55%. Ball burst data can be expressed as the ball burst strength as a function of mass per area of the sample; mass per area of the sample can be determined from the product of density and thickness of the sample.

Frazier Measurements

The Frazier permeability reading is the rate of flow of air in cubic feet per square foot of sample area per minute across the sample under a 12.7 mm water pressure. Air permeability was measured by clamping a test sample into a circular gasketed flanged fixture which provided a circular opening of 17.2 cm diameter (232 cm$^2$ area). The upstream side of the sample fixture was connected to a flow meter in line with a source of dry compressed air. The downstream side of the sample fixture was open to the atmosphere. The flow rate through the sample was measured and recorded as the Frazier number. The average of the three measurements was used.

Water Entry Pressure Measurement

Water entry pressure is a test method for measuring water intrusion through a membrane. A Mullen® Tester (Serial No: 8240 + 92 + 2949, manufactured by BF. Perkins, Chicopee, MA, USA) was used. A test sample was clamped between a pair of testing fixtures made of 1.27 cm thick square plexiglass sheets, 10.16 cm long on each side. The lower fixture had the ability to pressurize a section of the sample with water. A piece of pH paper was placed on top of the sample to serve as an indicator of evidence for water entry. The sample was then pressurized in small increments of pressure until a color change in the pH paper was noticed. The corresponding breakthrough pressure or entry pressure was recorded as the water entry pressure. The average of the three measurements was used.

Feature/Island Height Measurement

Feature, or island, height of the membranes surface treated with argon plasma was measured from scanning electron micrographs of longitudinal cross-sections of the samples. Individual values of island height were measured as the shortest distance from the node-fibril ePTFE structure to the highest point of the overlying feature. A line was drawn across the top surface of the node-fibril structure adjacent to the feature. A perpendicular line was then dropped from the highest point on the island to the line on the surface of the node-fibril structure.

The length of the dropped line is the island height. Measurements were preferably taken from micrographs taken at sufficiently high magnification to enable a clear determination of the height, taking into account the magnification of the scale bar at the bottom corner of the figure. Individual measurements were taken for five randomly chosen islands that were representative of all the islands. The reported island height value is the average of those five individual measurements.

Without intending to limit the scope of the present invention, the following examples illustrate how the present invention may be made and used.

## Example 1

An artificial cornea of the present invention was constructed in the following manner.

A random fluorinated copolymer consisting of approximately 50% (by wt) tetrafluoroethylene (TFE) and 50% (by wt) perfluoromethyl vinyl ether (PMVE) was made by emulsion polymerization, resulting in an average emulsion particle size of less than 100 nanometers (particle size estimated using light scattering methods), exhibiting the following properties: mean tensile strength of 31 MPa (+/- 8 MPa), mean 100% secant modulus of 3.7 MPa (+/- 0.5 MPa).

Approximately 14.2 g of polymer were placed in an approximately 1 ¾ inch (44.5 mm) diameter puck-shaped mold within a vacuum press. The polymer was then vacuum compressed into 1¾ inch (44.5 mm) pucks of approximately 4 mm thickness under a vacuum of 78 KPa, a temperature of about 250°C and under about 3.45 MPa pressure for about 6 minutes.

Subsequently, 5 mm diameter disks were punched from the pucks using a die cutter and used as the starting material for the molding process described in this Example. The weight of each disk of starting material was generally between 100-120 mg.

A disk was placed in a compression mold having substantially the geometry to form a shape as shown for the disk 12 in Figure 3. The mold was evacuated to 25-29 inches of mercury vacuum (85-100 MPa of vacuum) at room temperature, and then placed in a Carver press (Carver, Inc., Wabash, IN) with platens of 232 cm$^2$ cross-sectional area maintained at 220° C. The platens were brought in contact with the mold so as to apply minimal pressure on the mold (i.e., only contact of the plate with the mold to enable heating of the mold). The platen temperature setpoint was then changed to 200° C. The mold was held under these conditions for 25 minutes. At the end of 25 minutes, the heat to the platens was turned off and the platen pressure was increased to 4 metric tons and maintained during cooling. Air blowers were then used to rapidly cool the mold. Once the mold had reached room temperature, the resulting molded fluoropolymer optic disk was carefully removed from the mold. Any excess polymer "flash," or material overflow, was cut off during the molding process.

Expanded polytetrafluoroethylene (ePTFE) having a density of 0.4 (+/-0.02) g/cc, matrix tensile strength of about 14,000 psi (96 MPa) in two orthogonal directions, water entry pressure of 10.2 (+/-0.6) psi (70+/-4 KPa) and thickness of about 0.1 mm was employed as the first and second annular layers, each having an inner opening matching the anterior and posterior protrusions, respectively, of the disk.

The ePTFE employed in the annular layers was surface treated using an argon plasma. Only the side of the membrane to be exposed (i.e., the side which would face away from the disk of TFE/PMVE polymer) was surface treated with a hand-held plasma treater as described in accordance with the teaching of the U.S. patent application Publication No US 2006/0047311 (U.S. patent application Serial No. 11/000,414). The treated samples were heat treated unrestrained at 175° C. for 30 minutes in a convection oven. The surface treatment resulted in a morphology with features having an average feature height measurement of about 13-18 μm and a peak-to-valley distance of about 40-50 μm.

To form the annular layers of the device, ePTFE was then restrained in hoops, and holes corresponding to the anterior or posterior protrusion diameter were laser cut using a CO2 laser (Model ML-9370F, Keyence, Inc., NJ). The laser spot size and intensity were 60 μm and 30%, respectively, and the traversing speed of the laser was 200 mm/s. Specifically, for the annular layer to be oriented on the posterior surface of the polymeric corneal substitute material, the surface treated side was oriented downward, then a hole corresponding to the posterior protrusion was cut as described. Correspondingly, for the annular layer to be oriented on the anterior side of the polymeric corneal substitute material, the surface treated side was oriented upward, then a hole corresponding to the anterior protrusion was cut as described. The disk of polymeric corneal substitute material was then placed so that the posterior protrusion extended through the hole in the annular layer (treated surface facing downward). Subsequently, the cut membrane with treated surface facing upward was then oriented around the anterior protrusion of the polymeric corneal substitute material.

To form the sealing region of this example, an annular layer was made from expanded polytetrafluoroethylene (ePTFE) having a density of 0.4 (+/- 0.02) g/cc, matrix tensile strength of about 14,000 psi (96 MPa) in two orthogonal directions, water entry pressure of 10.2 (+/- 0.6) psi (70 +/- 4 KPa) and thickness of about 0.1 mm.

The ePTFE was laser cut using a $CO_2$ laser (Model ML-9370F, Keyence, Inc., NJ). The laser spot size and intensity were 60 $\mu$m and 27.5%, respectively, and the traversing speed of the laser was 200 mm/s. The size of the 2 cuts were for the inner diameter of the sealing portion adjacent to the optical surface and for the outer diameter (essentially the inner diameter of the anterior skirt). This annular ring was then placed on the sealing portion of the anterior surface adjacent to the optical surface of the polymeric corneal substitute material.

The assembled layers of polymeric corneal material, and ePTFE layers were then heated and compressed together in the following manner.

In a first step, the posterior protrusion of the polymeric corneal substitute material (assembled with ePTFE layers) was centrally oriented so as to rest on a high precision planar convex fused silica lens (Edmund Optics lens with 6 mm diameter (machined down to 4.5mm), +18 mm focal length). This allowed for shaping of the posterior protrusion to have a concave geometry. The ePTFE surfaces were then compressed by precision machined parts to apply 7 KPa using gravity. Here, the annular portion and the sealing region portion (anterior side) of the assembly were compressed independently. The entire assembly was then placed in a convection oven at 175°C for 45 minutes. The hot assembly was then removed from the oven and the precision machined part compressing the sealing region ring was replaced by a high precision planar concave fused silica lens (Edmund Optics lens with 6mm diameter, -18 mm focal length) and a weight to apply about 7 KPa pressure. In this step the precise optical surface of the anterior protrusion of convex curvature was formed by placing the assembly for another 25 minutes in the convection oven at 175°C. The assembly was subsequently cooled down to room temperature. The load and silica lenses were then removed, and the $CO_2$ laser was used to cut the outer diameter of the device to about 9.5 mm.

The keratoprosthesis was then treated using the following process:

1) The keratoprosthesis was immersed slowly edgewise into 100% isopropyl alcohol. This forced the residual air from the porous expanded PTFE, allowing the alcohol to fully penetrate the porous annular and sealing region layers.
2) The keratoprosthesis was then soaked in a 2% (wt/vol) polyvinyl alcohol (PVA)/deionized (DI) water solution for 15 minutes.
3) The keratoprosthesis was then rinsed in DI water for 15 minutes.
4) The keratoprosthesis was then placed in a 4% glutaraldehyde/2.6% hydrochloric acid (37.6% NF grade)/DI water solution (vol/vol/vol) for 15 minutes.
5) The keratoprosthesis was then rinsed in DI water for 15 minutes.
6) The treated keratoprosthesis was then air dried.

After hydrophilic treatment, the prototypes were steam sterilized at 121°C for 30 minutes prior to implantation.

## Implantation

The keratoprostheses were implanted in rabbits via a prosthokeratoplasty technique and sutured in place using Ethicon 10-0 CS160-6 suture (Ethicon Inc., Somerville, Inc.).

Performance evaluations of the implanted eye were performed first at 7 $\pm$ 2 days, then 14 $\pm$ 2 days, post-operatively and recorded. At 21 days post-implantation, the keratoprostheses were visually observed to show no sign of infection or extrusion and were tolerated by the animals.

## Example 2

An artificial cornea of the present invention was constructed in the following manner.

A disk was formed of the same material as described in Example 1 under the same processing conditions, with the only difference being that the shape of the disk was as shown in Figure 6.

Annular layers were made having the same composition and surface treatment as described in Example 1, and these layers were oriented on the anterior and posterior sides of the disk in the same manner as described in Example 1.

To form the sealing region (e.g., referred to as 36 in Figure 6) of this example, a thin circular disk of expanded polytetrafluoroethylene (ePTFE) was used for covering the optical surface. The ePTFE was made according to the teachings of U.S. Pat. No. 5,814,405 and had the following properties: mass per unit area of 1.7 $g/m^2$, thickness of 0.0003 inch (0.008 mm), density of 0.203 g/cc, longitudinal break load of 0.39 kg (0.85 lbs), ball burst strength of 0.65 kg (1.44 lbs) and Frazier number air flow of 18.4 $m^3/min/m^2$ (60.5 $cfm/ft^2$) at 1.27 cm (0.5 inch) $H_2O$ pressure.

The ePTFE membrane for the sealing region was laser cut using a $CO_2$ laser (Model ML-9370F, Keyence, Inc., NJ). The laser spot size and intensity were 60 $\mu$m and 22%, respectively, and the traversing speed of the laser was 200 mm/s. The size of the cut for the outer diameter was essentially the same as the inner diameter of the anterior annular ePTFE layer. This disk was then placed on the optical surface on the anterior protrusion of the polymeric corneal substitute material.

The assembled layers of polymeric corneal material, and ePTFE layers were then heated and compressed together in the following manner.

In a first step, the posterior protrusion of the polymeric corneal substitute material (assembled with ePTFE layers) was centrally oriented so as to rest on a high precision planar convex fused silica lens (Edmund Optics lens with 6 mm diameter (machined down to 4.5 mm), +18 mm focal length). This allowed for shaping of the posterior protrusion to have a concave geometry. The ePTFE skirts were then compressed by a precision machined part to apply 7 KPa pressure using gravity while a high precision planar concave fused silica lens (Edmund Optics lens with 6mm diameter, -18 mm focal length) and a weight applying about 7 KPa was placed on the ePTFE sealing disc. Here, the annular layer portion and the sealing region disc portion (ePTFE disc on the optical surface, anterior side) of the assembly were compressed independently. The entire assembly was then placed in a convection oven at 175°C for 60 minutes. The assembly was allowed to cool to room temperature. The load and silica lenses were then removed, and the $CO_2$ laser was used to cut the outer diameter of the device to about 9.5 mm.

The keratoprosthesis was then treated in the same manner as described in Example 1.

After drying, the prototypes were steam sterilized at 121°C for 30 minutes prior to implantation.

**Implantation**

The keratoprostheses were implanted in rabbits via a prosthokeratoplasty technique and sutured in place using Ethicon 10-0 CS160-6 suture (Ethicon Inc., Somerville, NJ).

Performance evaluations of the implanted eye were performed first at 7 $\pm$ 2 days, then 14$\pm$2 days, post-operatively and recorded. At 21 days post-implantation, the keratoprostheses were visually observed to show no sign of infection or extrusion and were tolerated by the animals.

**Example 3**

An artificial cornea device of the present invention is made in accordance with the teachings of Example 1, with the exception that both the sealing region and the annular rings have the same composition and surface treatment as the annular rings described in Example 1.

**Example 4**

An artificial cornea device of the present invention is made in accordance with the teachings of Example 1, with the exception that a plurality of openings, such as those shown in Figure 8a, are formed in the annular rings to create passageways, or through-paths, through the annular ring portion of the artificial cornea.

While particular embodiments of the present invention have been illustrated and described herein, the present invention should not be limited to such illustrations and descriptions. It should be apparent that changes and modifications may be incorporated and embodied as part of the present invention within the scope of the following claims.

**Claims**

**1.** An artificial cornea (10) comprising: a disk (12) comprising polymeric corneal substitute material having anterior and posterior surfaces, said disk having a protrusion (14) comprising an optical surface (18) on said anterior surface; a mechanical anchoring region comprising (1) an annular layer (20) having an inner opening (22), said annular layer comprising a polymeric material oriented on the anterior surface of said disk so that the inner opening of the annular layer is oriented around said protrusion, and (2) a second layer (21) comprising a polymeric material oriented on the posterior surface (15) of the disk; and a sealing region (28) around the perimeter of the optical surface, wherein said polymeric corneal substitute material comprises:

perfluoropolymer containing TFE as a comonomer; or
a fluoropolymer comprising a copolymer of tetrafluoroethylene (TFE) and perfluoroalkylvinylether (PAVE); or
a fluoropolymer comprising perfluoropolyether; or
a fluoropolymer comprising a copolymer of TFE and hexafluoropropylene (FEP).

2. An artificial cornea according to claim 1 wherein said disk has a first protrusion (14) comprising an optical surface (18) on said anterior surface and a second protrusion (16) on said posterior surface; a said mechanical anchoring region comprises (1) a first annular layer (20) having an inner opening (22), said first annular layer comprising a first polymeric material oriented on the anterior surface of said disk so that the inner opening of the first annular layer is oriented around said first protrusion, and (2) a second annular layer (24) having an inner opening (26), said second annular layer comprising a second polymeric material oriented on the posterior surface of said disk so that the inner opening of the second annular layer is oriented around said second protrusion.

3. The artificial cornea of claim 1 or 2, wherein said sealing region comprises:

   a porous material; or
   a non-porous material having at least some surface features thereon; or
   a porous material having at least some surface features thereon; or
   a porous material and at least a portion of the porosity is filled with a second material selected from the group consisting of a biodegradable material, a drug and a cell growth enhancer; or
   an annular sealing layer comprising a porous fluoropolymer; or
   a continuous layer across the optical surface; or
   expanded PTFE; or
   a continuous layer across the optical surface and wherein sealing region comprises expanded PTFE.

4. The artificial cornea of claim 1, wherein the annular layer and the sealing region comprise a single continuous material, or the artificial cornea of claim 2, wherein the first annular layer and the sealing region comprise a single continuous material.

5. The artificial cornea of claim 1 or 2, wherein said polymeric corneal substitute material:

   has a refractive index between about 1.2 and 1.6; or
   comprises at least one material selected from the group consisting of silicone, PMMA, hydrogel and polyurethane; or
   has a light transmission (visible wavelength) greater than 50%; or
   has a light transmission (visible wavelength) greater than 80%.

6. The artificial cornea of claim 1, wherein said annular layer and said second layer comprise expanded PTFE, or the artificial cornea of claim 2, wherein said first annular layer and said second layer comprise expanded PTFE

7. The artificial cornea of claim 1 or 2, further comprising:

   at least one reinforcing member (30) within at least a portion of said disk; or
   at least one through-path in the disk; or
   at least one through-path in the disk and wherein said at least one through-path extends through the disk and through the annular layer and the second layer of claim 1, or the first and second annular layers of claim 2.

8. An artificial cornea according to claim 2, wherein said first annular layer and second annular layer are porous.

9. An artificial cornea comprising: a disk comprising a polymeric corneal substitute material having anterior and posterior surfaces, said disk having on said anterior surface an optical surface; a mechanical anchoring means; and a sealing region around the perimeter of the optical surface, wherein said polymeric corneal substitute material comprises a fluoropolymer and wherein said fluoropolymer comprises at least one material selected from the group consisting of a copolymer of tetrafluoroethylene (TFE) and perfluoroalkylvinylether (PAVE), a copolymer of tetrafluoroethylene (TFE) and hexafluoropropylene (PAVE), and perfluoropolyether.

10. The artificial cornea of claim 9, wherein said sealing region comprises:

    an annular layer of a porous fluoropolymer; or
    a continuous layer across the optical surface; or
    expanded PTFE; or
    an annular layer of a porous fluoropolymer and expanded PTFE; or

a continuous layer across the optical surface and expanded PFTE; or
a non-porous material having at least some surface features thereon; or
a porous material having at least some surface features thereon; or
a porous material and at least a portion of the porosity is filled with a second material selected from the group consisting of a biodegradable material, a drug and a cell growth enhancer.

11. The artificial cornea of claim 9, wherein said polymeric corneal substitute material:

has a refractive index between about 1.2 and 1.6; or
comprises at least one material selected from the group consisting of silicone, PMMA, hydrogel and polyurethane; or
has a light transmission (wavelength 350-750 nm) greater than 50%; or
has a light transmission (wavelength 350-750 nm) greater than 80%.

12. The artificial cornea of claim 9, further comprising:

at least one reinforcing member (30) within at least a portion of said disk; or
at least one through-path in the disk.

13. The artificial cornea of claim 9, wherein said at least one through-path extends through the disk and through the mechanical anchoring means.


**Patentansprüche**

1. Künstliche Hornhaut (10), die Folgendes umfasst: eine Scheibe (12), die einen Polymer-Hornhautersatzwerkstoff umfasst, mit einer anterioren und einer posterioren Fläche, wobei die Scheibe einen Vorsprung (14) hat, der eine optische Oberfläche (18) auf der anterioren Fläche umfasst, einen mechanischen Verankerungsbereich, umfassend (1) eine ringförmige Lage (20), die eine innere Öffnung (22) hat, wobei die ringförmige Lage einen Polymerwerkstoff umfasst, der so auf der anterioren Fläche der Scheibe ausgerichtet ist, dass die innere Öffnung der ringförmigen Lage um den Vorsprung ausgerichtet ist, und (2) eine zweite Lage (21), die einen Polymerwerkstoff umfasst, der auf der posterioren Fläche (15) der Scheibe ausgerichtet ist, und einen Abdichtungsbereich (28) um den Umfang der optischen Oberfläche, wobei der Polymer-Hornhautersatzwerkstoff Folgendes umfasst:

ein Perfluorpolymer, das TFE als ein Comonomer enthält, oder
ein Fluorpolymer, das ein Copolymer aus Tetrafluorethylen (TFE) und Perfluoralkylvinylether (PAVE) umfasst, oder
ein Fluorpolymer, das Perfluorpolyether umfasst, oder
ein Fluorpolymer, das ein Copolymer von TFE und Hexafluorpropylen (FEP) umfasst.

2. Künstliche Hornhaut nach Anspruch 1, wobei die Scheibe einen ersten Vorsprung (14), der eine optische Oberfläche (18) auf der anterioren Fläche umfasst, und einen zweiten Vorsprung (16) auf der posterioren Fläche hat, wobei ein mechanischer Verankerungsbereich (1) eine erste ringförmige Lage (20), die eine innere Öffnung (22) hat, wobei die erste ringförmige Lage einen ersten Polymerwerkstoff umfasst, der so auf der anterioren Fläche der Scheibe ausgerichtet ist, dass die innere Öffnung der ersten ringförmigen Lage um den Vorsprung ausgerichtet ist, und (2) eine zweite ringförmige Lage (24), die eine innere Öffnung (26) hat, wobei die zweite ringförmige Lage einen zweiten Polymerwerkstoff umfasst, der so auf der anterioren Fläche der Scheibe ausgerichtet ist, dass die innere Öffnung der zweiten ringförmigen Lage um den Vorsprung ausgerichtet ist, umfasst.

3. Künstliche Hornhaut nach Anspruch 1 oder 2, wobei der Abdichtungsbereich Folgendes umfasst:

einen porösen Werkstoff, oder
einen nicht porösen Werkstoff, der wenigstens einige Oberflächenmerkmale auf demselben hat, oder
einen porösen Werkstoff, der wenigstens einige Oberflächenmerkmale auf demselben hat, oder
einen porösen Werkstoff, und wenigstens ein Teil der Porosität ist mit einem zweiten Werkstoff gefüllt, der ausgewählt ist aus der Gruppe, die aus einem biologisch abbaubaren Werkstoff, einem Arzneimittel und einem Zellwachstumsverstärker besteht, oder
eine ringförmige Abdichtungslage, die ein poröses Fluorpolymer umfasst, oder

eine durchgehende Lage über der optischen Oberfläche oder
geschäumtes PTFE oder
eine durchgehende Lage über der optischen Oberfläche und wobei der Abdichtungsbereich geschäumtes PTFE umfasst.

4. Künstliche Hornhaut nach Anspruch 1, wobei die ringförmige Lage und der Abdichtungsbereich einen einzigen durchgehenden Werkstoff umfassen, oder künstliche Hornhaut nach Anspruch 2, wobei die erste ringförmige Lage und der Abdichtungsbereich einen einzigen durchgehenden Werkstoff umfassen.

5. Künstliche Hornhaut nach Anspruch 1 oder 2, wobei der Polymer-Hornhautersatzwerkstoff:

einen Brechungskoeffizienten zwischen etwa 1,2 und 1,6 hat oder
wenigstens einen Werkstoff umfasst, der ausgewählt ist aus der Gruppe, die aus Silikon, PMMA, Hydrogel und Polyurethan besteht, oder
eine Lichtdurchlässigkeit (sichtbare Wellenlänge) von mehr als 50 % hat oder
eine Lichtdurchlässigkeit (sichtbare Wellenlänge) von mehr als 80 % hat.

6. Künstliche Hornhaut nach Anspruch 1, wobei die ringförmige Lage und die zweite Lage geschäumtes PTFE umfassen, oder künstliche Hornhaut nach Anspruch 2, wobei die erste ringförmige Lage und die zweite Lage geschäumtes PTFE umfassen.

7. Künstliche Hornhaut nach Anspruch 1 oder 2, die ferner Folgendes umfasst:

wenigstens ein Verstärkungselement (30) innerhalb wenigstens eines Abschnitts der Scheibe oder
wenigstens eine Durchgangsbahn in der Scheibe oder
wenigstens eine Durchgangsbahn in der Scheibe und wobei sich die wenigstens eine Durchgangsbahn durch die Scheibe und durch die ringförmige Lage und die zweite Lage von Anspruch 1 oder die erste und die zweite ringförmige Lage von Anspruch 2 erstreckt.

8. Künstliche Hornhaut nach Anspruch 2, wobei die erste ringförmige Lage und die zweite ringförmige Lage porös sind.

9. Künstliche Hornhaut, die Folgendes umfasst: eine Scheibe, die einen Polymer-Hornhautersatzwerkstoff umfasst, mit einer anterioren und einer posterioren Fläche, wobei die Scheibe auf der anterioren Fläche eine optische Oberfläche hat, ein mechanisches Verankerungsmittel und einen Abdichtungsbereich um den Umfang der optischen Oberfläche,
wobei der Polymer-Hornhautersatzwerkstoff ein Fluorpolymer umfasst und das Fluorpolymer wenigstens einen Werkstoff umfasst, der ausgewählt ist aus der Gruppe, die aus einem Copolymer aus Tetrafluorethylen (TFE) und Perfluoralkylvinylether (PAVE), einem Copolymer von Tetrafluorethylen (TFE) und Hexafluorpropylen (FEP) und Perfluorpolyether besteht.

10. Künstliche Hornhaut nach Anspruch 9, wobei der Abdichtungsbereich Folgendes umfasst:

eine ringförmige Abdichtungslage aus einem porösen Fluorpolymer oder
eine durchgehende Lage über der optischen Oberfläche oder
geschäumtes PTFE oder
eine ringförmige Lage aus einem porösen Fluorpolymer und geschäumtem PTFE oder
eine durchgehende Lage über der optischen Oberfläche und geschäumtes PTFE oder
einen nicht porösen Werkstoff, der wenigstens einige Oberflächenmerkmale auf demselben hat, oder
einen porösen Werkstoff, der wenigstens einige Oberflächenmerkmale auf demselben hat, oder
einen porösen Werkstoff, und wenigstens ein Teil der Porosität ist mit einem zweiten Werkstoff gefüllt, der ausgewählt ist aus der Gruppe, die aus einem biologisch abbaubaren Werkstoff, einem Arzneimittel und einem Zellwachstumsverstärker besteht.

11. Künstliche Hornhaut nach Anspruch 9, wobei der Polymer-Hornhautersatzwerkstoff:

einen Brechungskoeffizienten zwischen etwa 1,2 und 1,6 hat oder
wenigstens einen Werkstoff umfasst, der ausgewählt ist aus der Gruppe, die aus Silikon, PMMA, Hydrogel und Polyurethan besteht, oder

eine Lichtdurchlässigkeit (Wellenlänge 350-750 mm) von mehr als 50 % hat oder
eine Lichtdurchlässigkeit (Wellenlänge 350-750 mm) von mehr als 80 % hat.

12. Künstliche Hornhaut nach Anspruch 9, die ferner Folgendes umfasst:

wenigstens ein Verstärkungselement (30) innerhalb wenigstens eines Abschnitts der Scheibe oder
wenigstens eine Durchgangsbahn in der Scheibe.

13. Künstliche Hornhaut nach Anspruch 9, wobei sich die wenigstens eine Durchgangsbahn durch die Scheibe und durch das mechanische Verankerungsmittel erstreckt.


**Revendications**

1. Cornée artificielle (10), comprenant : un disque (12), comprenant un matériau de substitut cornéen polymère comportant des surfaces antérieure et postérieure, ledit disque comportant une saillie (14), comprenant une surface optique (18) sur ladite surface antérieure, une région d'ancrage mécanique, comprenant (1) une couche annulaire (20) comportant une ouverture interne (22), ladite couche comprenant un matériau polymère orienté sur la surface antérieure dudit disque, de sorte que l'ouverture interne de la couche annulaire est orientée autour de ladite saillie, et (2) une deuxième couche (21), comprenant un matériau polymère orienté sur la surface postérieure (15) du disque; et une région d'étanchéité (28) entourant le périmètre de la surface optique, ledit matériau de substitut cornéen polymère comprenant :

un polymère perfluoré, contenant du tétrafluoroéthylène comme comonomère ;
un polymère fluoré, comprenant un copolymère de tétrafluoroéthylène (TFE) et du perfluoroalkylvinyléther (PAVE) ; ou
un polymère fluoré comprenant un perfluoropolyéther ; ou
un polymère fluoré comprenant un polymère de tétrafluoroéthylène et de hexafluoropropylène (FEP).

2. Cornée artificielle selon la revendication 1, dans laquelle ledit disque comporte une première saillie (14), comprenant une surface optique (18) sur ladite surface antérieure, et une deuxième saillie (16) sur ladite surface postérieure ; une dite région d'ancrage mécanique comprenant (1) une première couche annulaire (20), comportant une ouverture interne (22), ladite première couche annulaire comprenant un premier matériau polymère orienté sur la surface antérieure dudit disque, de sorte que l'ouverture interne de la première couche annulaire est orientée autour de ladite première saillie, et (2) une deuxième couche annulaire (24), comprenant une ouverture intérieure (26), la deuxième couche annulaire comprenant un deuxième matériau polymère orienté sur la surface postérieure dudit disque, de sorte que l'ouverture interne de la deuxième couche annulaire est orientée autour de ladite deuxième saillie.

3. Cornée artificielle selon les revendications 1 ou 2, dans laquelle ladite région d'étanchéité comprend :

un matériau poreux ; ou
un matériau non poreux, comportant au moins certaines structures de surface qui y sont agencées; ou
un matériau poreux, comportant au moins certaines structures de surfaces qui y sont agencées ; ou
un matériau poreux, au moins une partie de la porosité étant remplie d'un deuxième matériau sélectionné dans le groupe constitué d'un matériau biodégradable, d'un médicament et d'un stimulateur de la croissance cellulaire ; ou
une couche d'étanchéité annulaire, comprenant un polymère fluoré poreux ; ou
une couche continue à travers la surface optique ; ou
du PTFE expansé ; ou
une couche continue à travers la surface optique, la région d'étanchéité comprenant du PTFE expansé.

4. Cornée artificielle selon la revendication 1, dans laquelle la couche annulaire et la région d'étanchéité comprennent un seul matériau continu, ou cornée artificielle selon la revendication 2, dans laquelle la première couche annulaire et la région d'étanchéité comprennent un seul matériau continu.

5. Cornée artificielle selon les revendications 1 ou 2, dans laquelle ledit matériau de substitut cornéen polymère :

présente un indice de réfraction compris entre environ 1,2 et 1,6 ; ou

comprend au moins un matériau sélectionné dans le groupe constitué de silicone, de PMMA, d'hydrogel et de polyuréthane ; ou

présente une transmission de la lumière (longueur d'onde visible) supérieure à 50% ; ou

présente une transmission de la lumière (longueur d'onde visible) supérieure à 80%.

6. Cornée artificielle selon la revendication 1, dans laquelle ladite couche annulaire et ladite deuxième couche comprennent du PTFE expansé, ou cornée artificielle selon la revendication 2, dans laquelle ladite première couche annulaire et ladite deuxième couche comprennent du PTFE expansé.

7. Cornée artificielle selon les revendications 1 ou 2, comprenant en outre :

au moins un élément de renforcement (30) dans au moins une partie dudit disque ; ou

au moins un trajet traversant dans le disque ; ou

au moins un trajet traversant dans le disque, ledit au moins un trajet traversant s'étendant à travers le disque et à travers la couche annulaire et la deuxième couche selon la revendication 1, ou les première et deuxième couches annulaires selon la revendication 2.

8. Cornée artificielle selon la revendication 2, dans laquelle ladite première couche annulaire et ladite deuxième couche annulaire sont poreuses.

9. Cornée artificielle, comprenant : un disque, comprenant un matériau de substitut cornéen polymère, comportant des surfaces antérieure et postérieure, ledit disque comportant une surface optique sur ladite surface antérieure ; un moyen d'ancrage mécanique ; et une région d'étanchéité entourant le périmètre de la surface optique ; ledit matériau de substitut cornéen polymère comprenant un polymère fluoré, ledit polymère fluoré comprenant au moins un matériau sélectionné dans le groupe constitué d'un copolymère de tétrafluoroéthylène (TFE) et de perfluoroalkylvinyléther (PAVE), d'un copolymère de tétrafluoroéthylène (TFE) et d'hexafluoropropylène (FEP), et d'un polyéther perfluoré.

10. Cornée artificielle selon la revendication 9, dans laquelle ladite région d'étanchéité comprend :

une couche annulaire d'un polymère fluoré poreux ; ou

une couche continue à travers la surface optique ; ou

du PTFE expansé ; ou

une couche annulaire d'un polymère fluoré poreux et de PTFE expansé ; ou

un matériau non poreux, comportant au moins certaines structures de surface qui y sont agencées ; ou

un matériau poreux, au moins une partie de la porosité étant remplie d'un deuxième matériau sélectionné dans le groupe constitué d'un matériau biodégradable, d'un médicament et d'un stimulateur de la croissance cellulaire.

11. Cornée artificielle selon la revendication 9, dans laquelle ledit matériau de substitut cornéen polymère :

présente un indice de réfraction compris entre environ 1,2 et 1,6 ; ou

comprend au moins un matériau sélectionné dans le groupe constitué de silicone, de PMMA, d'hydrogel et de polyuréthane ; ou

présente une transmission de la lumière (longueur d'onde 350 - 750 nm) supérieure à 50% ; ou

présente une transmission de la lumière (longueur d'onde 350 - 750 nm) supérieure à 80%.

12. Cornée artificielle selon la revendication 9, comprenant en outre :

au moins un élément de renforcement (30) dans au moins une partie dudit disque ; ou

au moins un trajet traversant dans le disque.

13. Cornée artificielle selon la revendication 9, dans laquelle ledit au moins un trajet traversant s'étend à travers le disque et à travers le moyen d'ancrage mécanique.

**FIG. 1a**

EP 2 136 737 B1

**FIG. 1b**

FIG. 2

**FIG. 3**

EP 2 136 737 B1

EP 2 136 737 B1

**FIG. 4**

**FIG. 5**

EP 2 136 737 B1

FIG. 6

FIG. 7

**FIG. 8a**

FIG. 8b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5282851 A, LaBarre
- US 5843185 A, Leon Rolden
- US 5489301 A, Barber
- US 6391055 B, Ikada
- US 5300116 A, Chirila
- US 6976997 B, Noolandi
- US 20040024448 A
- WO 20050129735 A
- WO 9727824 A
- US 20060047311 A
- US 000414 A
- US 5814405 A